# EUROPEAN PATENT SPECIFICATION

(11) **EP 0 588 127 B1**
(45) Date of publication and mention of the grant of the patent: **10.02.1999**
(21) Application number: 93113743.4
(22) Date of filing: 27.08.1993
(51) Int. Cl.: A61N 1/39

(54) **Implantable heart defibrillator**
Implantierbares Herz-Defibrillationsgerät
Defibrillateur cardiaque implantable

(30) Priority: 16.09.1992 SE 9202664
(43) Date of publication of application: 23.03.1994
(73) Proprietor: Pacesetter AB, 175 84 Järfälla (SE)
(72) Inventor: Hedberg, Sven-Erik, S-196 32 Kungsängen (SE); Obel, Martin, S-182 33 Danderyd (SE); Högnelid, Kurt, S-137 38 Västerhaninge (SE)
(74) Representative: Lettström, Richard Wilhelm

(56) References cited:
- US-A- 4 708 145
- US-A- 4 768 512
- US-A- 4 969 463
- US-A- 5 107 834

## Description

The present invention relates to an implantable heart defibrillator comprising a pulse generator, controlled by a control unit, for emitting defibrillation pulses.

In DE-A1-3 715 822, a cardioversion system is described in which the cardioversion pulse is chopped into a sequence of brief pulses in order to save energy. The general waveform of the pulse package emitted in this way describes the conformation of an exponentially decaying conventional cardioversion pulse, i.e. the amplitude of the individual pulses in the pulse package produced by chopping is the same on every occasion as in a corresponding, conventional cardioversion pulse.

In US-A-5 107 834 a defibrillation system is described where defibrillation pulses of a lower energy than a conventional defibrillation pulse are used. According to this patent it is necessary to use at least two pair of defibrillation electrodes, a first shock being delivered at an energy level lower than that typically necessary to cardiovert/defibrillate the entire heart and being applied between a first pair of electrodes and a second shock of an energy less than the first shock being applied between a second pair of electrodes to depolarize a particular area of the myocardium experiencing a low voltage gradient resulting from the first shock

The problem to be overcome by the invention is to achieve an implantable heart defibrillator which can effectively defibrillate the heart at a lower energy than is normally used, without necessitating several pairs of electrodes. Using a lower energy saves both the heart tissue and the defibrillator battery.

The heart is anisotropic in such a way that e.g. a defibrillation pulse excites heart cells more readily if the cells have a given direction in relation to the defibrillating electrical field. The electrical field strength required to excite a heart cell can vary between two orthogonal directions by a factor of 2 to 5.

The object of the present invention is to utilize the above-mentioned anisotropy of the heart to achieve heart defibrillation with lower energy than has hitherto been possible.

This object is achieved with a defibrillator of the above-described type with the characteristics cited in claim 1.

Thus, the defibrillator according to the invention emits a plurality of defibrillation pulses with energies which are less than the energy required to defibrillate the entire heart but which depolarize most non-refractory heart cells with a given direction in relation to the defibrillating electrical field. Since the heart cells are able to excite other cells for a rather long period of time, the aim is to prevent neighboring cells from being excited, when they leave their refractory phase, and propagate the signal. Even when it is not possible to prevent neighboring cells from becoming excited, it is possible to excite the next cell with a low-energy defibrillation pulse and, by emission of a plurality of such pulses, prevent cells leaving their refractory phase from being re-excited and becoming refractory. The emission for a period up to about the duration of one refractory period of a plurality of relatively low energy pulses, whose energy is still sufficient to depolarize heart cells in the most favourable direction during different phases of the depolarization wave's propagation, thereby stops propagation of a wave in the direction favourable at any given moment.

Thus, heart defibrillation with the aid of a plurality of low-energy defibrillation pulses, whose total energy is less than the energy in a conventional defibrillation shock or pulse, is achieved with the defibrillator according to the invention.

According to an advantageous embodiment of the defibrillator according to the invention, the pulse generator is devised to emit defibrillation pulses with a lower pulse amplitude and shorter pulse duration, each such pulse then consisting of a burst of impulses. This then prolongs the refractory period of certain heart cells, so an increasing number of heart cells become refractory and the prospects of terminating fibrillation increases.

The invention will now be described below in greater detail with the aid of an exemplified embodiment of the defibrillator according to the invention which is illustrated in the accompanying drawings.

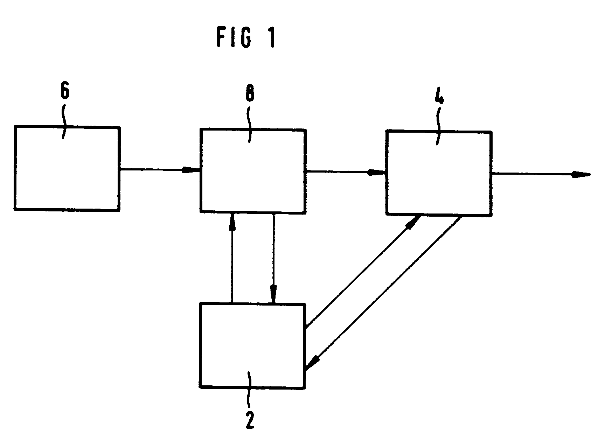

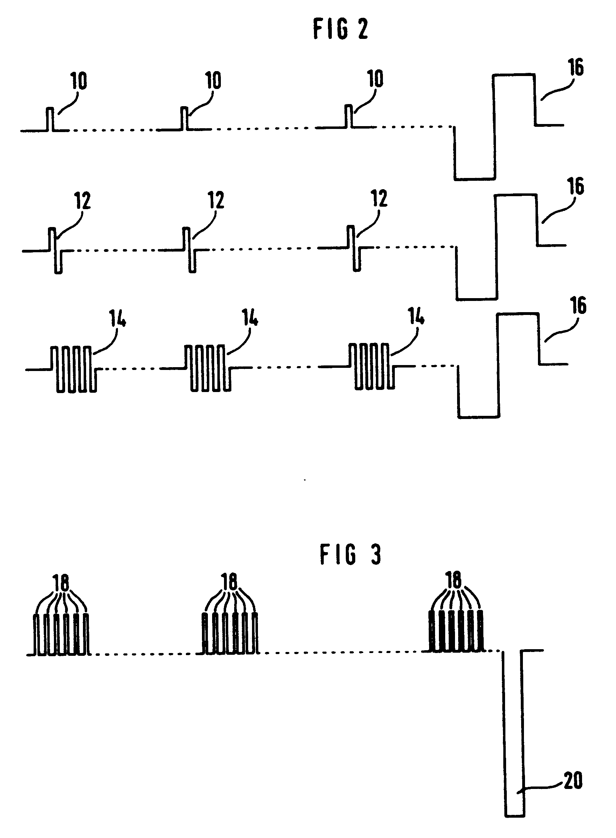

FIG. 1 shows, in block diagram form, a defibrillator according to the invention and FIGS. 2 and 3 show examples of sequences of low-energy defibrillation pulses, followed by a conventional defibrillation pulse, which can be achieved with the defibrillator according to the invention.

The defibrillator shown in FIG. 1 comprises a sensor with an associated amplifier 6 which senses and amplifies signals from the heart. The amplified sensor signal is analyzed in the analyzer section 8 to ascertain whether heart fibrillation is present.

The results of the analysis are sent to the control unit 2 which, when fibrillation is detected, induces the pulse generator 4 to send a plurality of defibrillation pulses with a lower pulse amplitude and shorter pulse duration than an ordinary defibrillation pulse to the defibrillation electrodes.

The pulse generator 4 contains an output stage (not shown) with switches controlled here by a microprocessor in the control unit 2, so pulse trains with desired conformation are emitted.

Pulse amplitude and pulse width can be set as desired by programming the microprocessor in the control unit 2.

The defibrillator according to the invention can be devised to emit monophasic or biphasic defibrillation pulses or combinations of such pulses.

FIG. 2 shows three examples of sequences of low-energy defibrillation pulses 10, 12, 14 with considerably lower amplitude and shorter pulse duration than a following, conventional defibrillation pulse 16.

In this example, the pulse generator 4 contains a requisite source of power and a high-voltage generator to emit conventional defibrillation pulses with a voltage of the order of 500 - 1,000 V and an energy content of ten joules or so. However, with a defibrillator according to the invention, effective heart defibrillation can be achieved solely with the low-energy defibrillation pulses 10, 12, 14.

The first sequence of low-energy defibrillation pulses shown in FIG. 2 comprises monophasic pulses 10, whereas the following pulse sequences consist of biphasic pulses 12, 14. The third pulse sequence in FIG. 2 consists of low-energy defibrillation pulses 14 arrayed in separate groups.

The total energy of the low-energy defibrillation pulses is considerably less than the energy in a conventional defibrillation pulse 16.

The distance between the low-energy defibrillation pulses 10, 12, 14 can vary from about one millisecond to about a hundred milliseconds, preferably of the order of ten milliseconds.

Such groups of low-energy defibrillation pulses can comprise 2 - 10 pulses.

The low-energy defibrillation pulses can be emitted for a period from about ten milliseconds up to about the duration of one refractory period or somewhat more.

In the case a strong, conventional defibrillation pulse 16 is emitted after the low-energy defibrillation pulses, the time for emission of the conventional defibrillation pulse can be selected from 0 seconds, i.e. the conventional defibrillation pulse 16 is emitted immediately after the last low-energy defibrillation pulse 10, 12, 14, to several seconds after the last low-energy defibrillation pulse.

FIG. 3 illustrates an embodiment in which each low-energy defibrillation pulse consists of a burst of impulses 18. In this way, the refractory time of certain cells can be prolonged, making more heart cells refractory and increasing the likelihood of terminating the fibrillation. In the example shown in FIG. 3, the low-energy defibrillation pulses formed by the impulses 18 are also followed by a conventional, strong defibrillation pulse 20, thereby reducing the energy saved.

The conformation of the pulses is unimportant to the function of the defibrillator according to the invention but can obviously be varied in many ways.

## Claims

1. An implantable heart defibrillator comprising a pulse generator (4) having one pair of electrodes and being controlled by a control unit (2) for emitting a plurality of defibrillation pulses for a period up to about the duration of one refractory period, where each pulse contains an energy which is less than the energy required to defibrillate the entire heart, but enough to depolarize most refractory heart cells with a favorable direction in relation to the direction of the defibrillating electrical field, and where the total energy of the pulses is less than the energy of a conventional defibrillation pulse which can defibrillate the entire heart.

2. The defibrillator of claim 1, characterized in that the pulse generator (4) is induced to emit said defibrillation pulses with a lower pulse amplitude and shorter pulse duration with an inter-pulse distance of from about one millisecond to about one hundred milliseconds, preferably of the order of ten milliseconds.

3. The defibrillator of claim 1 or 2, characterized in that the pulse generator (4) is induced to emit said defibrillation pulses with lower amplitude and shorter pulse duration in sequences of 2 - 10 such pulses.

4. The defibrillator of any of claims 1 - 3, characterized in that the pulse generator (4) is devised to emit monophasic or biphasic defibrillation pulses or combinations of monophasic or biphasic defibrillation pulses.

5. The defibrillator of any of claims 1 - 4, characterized in that the pulse generator (4) is devised to emit defibrillation pulses with a lower pulse amplitude and shorter pulse duration, each such pulse consisting of a burst of impulses.

6. The defibrillator of any of claims 1 - 5, characterized in that the pulse generator (4) contains an output stage for emitting defibrillation pulses with switches controlled by the control unit (2).

7. The defibrillator of any of claims 1 - 6, characterized in that the pulse generator (4) is induced to emit a conventional defibrillation pulse after a group of said defibrillation pulses of lower pulse amplitude and shorter pulse duration.

8. The defibrillator of claim 7, characterized in that the pulse generator (4) is induced to emit the conventional defibrillation pulse within a period of time ranging from 0 to several seconds after the last pulse in the group of defibrillation pulses with a lower pulse amplitude and shorter pulse duration.

## Patentansprüche

1. Ein implantierbarer Herzdefibrillator mit einem Impulsgenerator (4) mit einem Paar von Elektroden, der durch eine Steuereinheit (2) zur Abgabe einer Mehrzahl von Defibrillationsimpulsen für eine Dauer bis zu etwa der Dauer einer Refraktärperiode gesteuert wird, wobei jeder Impuls eine Energie enthält, die kleiner ist als die zum Defibrillieren des gesamten Herzens erforderliche Energie aber ausreichend, um die meisten refraktären Herzzellen mit einer bevorzugten Richtung in Bezug auf die Richtung des defibrillierenden elektrischen Feldes zu depolarisieren, und wobei die gesamte Energie der Impulse kleiner ist als die Energie eines konventionellen Defibrillationsimpulses, der das gesamte Herz defibrillieren kann.

2. Der Defibrillator nach Anspruch 1, **dadurch gekennzeichnet,** daß der Impulsgenerator (4) dazu angeregt wird, die Defibrillationsimpulse mit einer niedrigeren Impulsamplitude und kürzerer Impulsdauer mit einem Abstand zwischen den Impulsen von etwa einer Millisekunde bis zu etwa hundert Millisekunden, vorzugsweise in der Größenordnung von zehn Millisekunden abzugeben.

3. Der Defibrillator nach Anspruch 1 oder 2, **dadurch gekennzeichnet,** daß der Impulsgenerator (4) dazu angeregt wird, diese Defibrillationsimpulse mit niedrigerer Amplitude und kürzerer Impulsdauer in Sequenzen von 2 - 10 Impulsen abzugeben.

4. Der Defibrillator nach einem der Ansprüche 1 - 3, **da-durch gekennzeichnet,** daß der Impulsgenerator (4) so ausgebildet ist, um monophasische oder biphasische Defibrillationsimpulse oder Kombinationen von monophasischen oder biphasischen Impulsen abzugeben.

5. Der Defibrillator nach einem der Ansprüche 1 - 4, **dadurch gekennzeichnet,** daß der Impulsgenerator (4) so ausgebildet ist, um Defibrillationsimpulse mit einer niedrigeren Impulsamplitude und kürzerer Impulsdauer abzugeben, wobei jeder dieser Impulse aus einen Bündel von Impulsen besteht.

6. Der Defibrillator nach einem der Ansprüche 1 - 5, **dadurch gekennzeichnet,** daß der Impulsgenerator (4) eine Ausgangsstufe zur Abgabe von Defibrillationsimpulsen mit durch die Steuereinheit (2) gesteuerten Schaltern aufweist.

7. Der Defibrillator nach einem der Ansprüche 1 - 6, **da-durch gekennzeichnet,** daß der Impulsgenerator (4) dazu angeregt wird, einen konventionellen Defibrillationsimpuls nach einer Gruppe der Defibrillationsimpulse niedrigerer Amplitude und kürzerer Impulsdauer abzugeben.

8. Der Defibrillator nach Anspruch 7, **dadurch gekennzeichnet,** daß der Impulsgenerator (4) dazu angeregt wird, den konventionellen Defibrillationsimpuls innerhalb einer von 0 bis zu mehreren Sekunden nach dem letzten Impuls in der Gruppe von Defibrillationsimpulsen mit niedrigerer Amplitude und kürzerer Impulsdauer umspannenden Zeitdauer abzugeben.

## Revendications

1. Défibrillateur cardiaque implantable, comprenant un générateur (4) d'impulsion ayant un couple d'électrodes et commandé par une unité (2) de commande en vue d'émettre une pluralité d'impulsions de défibrillation pendant un laps de temps allant jusqu'à environ la durée d'une période réfractaire, chaque impulsion contenant une énergie qui est inférieure a l'énergie requise pour défibriller tout le coeur, mais suffisante pour dépolariser la plupart des cellules cardiaques réfractaires avec une direction favorable par rapport à la direction du champ électrique de défibrillation, et l'énergie totale des impulsions est inférieure à l'énergie d'une impulsion classique de défibrillation, qui peut défibriller tout le coeur.

2. Défibrillateur suivant la revendication 1, caractérisé en ce que le générateur (4) d'impulsions est amené a émettre les impulsions de défibrillation ayant une petite amplitude d'impulsion et une courte durée d'impulsion avec une distance entre les impulsions d'environ une milliseconde à environ cent de millisecondes, de préférence de l'ordre de 10 millisecondes.

3. Défibrillateur suivant la revendication 1 ou 2, caractérisé en ce que le générateur (4) d'impulsions est amené à émettre les impulsions de défribillation ayant une petite amplitude et une courte durée d'impulsion en séquence de deux à dix impulsions de ce genre

4. Défibrillateur suivant l'une quelconque des revendications 1 à 3, caractérisé en ce que le générateur (4) d'impulsions est constitué de manière à émettre des impulsions de défibrillation monophasées ou biphasées ou des combinaisons d'impulsion de défibrillation monophasées ou biphasées.

5. Défibrillateur suivant l'une quelconque des revendications 1 à 4, caractérisé en ce que le générateur (4) d'impulsions est constitué de manière à émettre des impulsions de défibrillation ayant une petite amplitude d'impulsion et une courte durée d'impulsion, chaque impulsion de ce genre consistant en une rafale d'impulsions.

6. Défibrillateur suivant l'une quelconque des revendications 1 à 5, caractérisé en ce que le générateur (4) d'impulsions comporte un étage de sortie pour émettre des impulsions de défibrillation, ayant des interrupteurs commandés par l'unité (2) de commande.

7. Défibrillateur suivant l'une quelconque des revendications 1 à 6, caractérisé en ce que le générateur (4) d'impulsions est amené à émettre une impulsion classique de défibrillation après un groupe desdites impulsions de défibrillation de petite amplitude d'impulsion et de courte durée d'impulsion.

8. Défibrillateur suivant la revendication 7, caractérisé en ce que le générateur (4) d'impulsion est amené à émettre l'impulsion classique de défibrillation en un laps de temps allant de 0 à plusieurs secondes, après la dernière impulsion du groupe des impulsions de défibrillation ayant une petite amplilude d'impulsion et une courte durée d'impulsion.
